## Europäisches Patentamt

## European Patent Office

## Office européen des brevets

(19)

(11) Publication number: **0 229 521**
**B1**

(12)

# EUROPEAN PATENT SPECIFICATION

(45) Date of publication of the patent specification:
06.12.89

(51) Int. Cl.⁴: **C 07 C 7/12,** C 08 F 236/04,
C 08 J 11/02

(21) Application number: **86310046.7**

(22) Date of filling: **22.12.86**

(54) **Reducing contamination of olefinic hydrocarbons.**

(30) Priority: **27.12.85 US 814097**

(43) Date of publication of application:
**22.07.87 Bulletin 87/30**

(45) Publication of the grant of the patent:
**06.12.89 Bulletin 89/49**

(84) Designated Contracting States:
**BE FR GB**

(56) References cited:
**GB-A-2 097 421**
**US-A-3 864 243**

(73) Proprietor: **EXXON CHEMICAL PATENTS INC.,
1900 East Linden Avenue, Linden New Jersey
07036 (US)**

(72) Inventor: **Atwood, Harvey Emerson, 5219
Sycamore Creek, Kingwood Texas (US)**

(74) Representative: **Dew, Melvyn John, Exxon
Chemical Limited Exxon Chemical Technology
Centre P.O. Box 1, Abingdon Oxfordshire, OX13
6BB (GB)**

LIBER, STOCKHOLM 1989

## Description

This invention relates to a method of decontaminating olefinic hydrocarbons, and is particularly but not exclusively concerned with such method when employed as part of a butyl rubber production process.

Butyl rubber was one of the earlier synthetic rubbers to be developed. It is a copolymer of isobutylene and a conjugated multiolefin, usually isoprene. It was long recognized that the isoprene could not be recycled in the butyl rubber process because the recycled monomer stream acted as a poison to the polymerization process. It was not until after a substantial number of years of discarding isoprene that the cause of this poisoning effect was understood. It is known to us that the poisoning effect is the result of the formation of t-butyl chloride (t-BCl) from isobutylene reactions with HCl. The t-butyl chloride tends to concentrate by fractionation in the recycle isoprene stream and affects the polymerization by chain transfer mechanisms so that lower molecular weight copolymers are produced.

Since t-butyl chloride and isoprene cannot easily be separated from one another by standard fractionation techniques, butyl rubber plants continue to discard the unreacted isoprene which has been used in the butyl rubber process rather than recycling it. The result is a significant increase in polymerization costs. For example, in a 50, 000 ton/yr. butyl rubber plant, isoprene recovery would reduce isoprene raw material costs by about $600,000 per year.

Many techniques are known for removing halides from process streams. For example, in the production of methyl chloride and methylene chloride by the (oxy) chlorination of methane, the chlorides can be recovered by gas phase adsorption in beds of adsorbed materials including silica gels, activated carbon, activatated alumina, molecular sieves, or their combinations; see U.S. Patent No. 4 020 117. The adsorption is carried out at about -50°C to about 20°C. The adsorbed halides are stripped from the adsorption stage at about 100 - 400°C. Similarly, German patent No. 2 839 516 discloses a process for purifying an exhaust gas stream to remove contaminants such as halogens or halogenated hydrocarbons by passing the gas through alumina or calcium compounds.

British patent No. 1 438 246 discloses a process for reacting a chloroform process stream containing impurities by contacting the stream in the vapor phase with activated carbon or alumina. It is alleged that $CH_2ClBr$ which is present as an impurity in the chloroform, reacts with the chloroform to form $CHCl_2Br$ and $CH_2Cl_2$ which are then readily separated from the chloroform by distillation.

Soviet Union patent No. 506 597 teaches the purification of recycled methylene chloride-isobutylene stream by passing the compounds first in the vapor phase over alumina and then in the liquid phase at 10° to 20°C. It is disclosed that the process removes water, dimethyl ether, and HCl from the stream.

U.S.Patent No. 2 347 945 discloses a method for removing organic fluorides from a hydrocarbon stream either in the liquid or gaseous phase by contacting the scream with a "contact material." The contact material can be alumina, hydrated bauxite, chromium oxide, and metals from the iron groups, especially nickel deposited on an inert support.

U.S. Patent No. 3 864 243 discloses a process for the removal of combined chlorine (organic or inorganic) from a hydrocarbon stream by percolating the hydrocarbon through a bed of dehydrated activated alumina, e.g. bauxite. The adsorption process is said to be more effective at room temperature than at elevated temperatures, e .g., 98°C. Similarly, U.S. Patent No. 3 862 900 discloses the room temperature adsorption of organic halides on molecular sieves (pore size 7 - 11 A).

U.S. Patent No. 2 412 220 discloses a process for the removal or organic fluorides from a hydrocarbon stream by passing the hydrocarbon through a bed of alumina which is catalytically active for hydrogenation or dehydrogenation. It is alleged that the effluent stream contains silicon fluorides which are subsequently removed by treating the hydrocarbon stream with an alkali metal hydroxide, e.g., NaOH, and then filtering the hydrocarbon stream through a non-siliceous granular filter medium, e.g., charcoal. In a similar vein, U.S. Patent No. 2 391 149 discloses the removal of fluorides from a hydrocarbon stream by contacting the hydrocarbon with alumina which has been impregnated with an alkali metal hydroxide.

While the art generally teaches the use of materials such as activated carbon and alumina for the purification of halide containing process streams, it is apparent from these disclosures that not all organic halides are removed from a process stream contacted with these and other materials of the prior art. Furthermore, there is no disclosure of the removal of t-butyl chloride from such hydrocarbon streams; nor is there any teaching from which it could be concluded that a particular contact medium is preferred over others for the removal of t-butyl chloride from an isoprene stream.

According to one aspect of the present invention there is provided a method of treating an olefinic hydrocarbon contaminated with tertiary butyl chloride, to reduce the contamination level thereof, which method comprises contacting the contaminated hydrocarbon with activated alumina for a time sufficient to achieve such reduction.

In one embodiment of the method the contaminated olefinic hydrocarbon comprises unreacted dried monomer(s) separated from the product mixture of a methyl chloride slurry polymerisation process for preparing butyl rubber. Such monomers may comprise isoprene which has been separated from unreacted dried isobutylene comonomer by fractionation and thereby enriched in tertiary butyl chloride. The treated hydrocarbon may be recycled to the polymerisation process.

It has surprisingly been found that a recovery stream of isoprene from a butyl rubber polymerisation process can be purified by treatment with alumina. The resulting treated isoprene can be recycled for use in the butyl rubber process.

In a preferred embodiment, the hydrocarbon stream is contacted with the alumina at a temperature of 40°C to 80°C. Surprisingly, the elevated temperature results in an improvement in t-butyl chloride removal.

Butyl rubber is a copolymer of an isoolefin and a conjugated multiolefin. The useful copolymers comprise a major portion of an isoolefin and a minor amount, preferably not more than 30 wt%, of a conjugated multiolefin. The preferred copolymers comprise about 85 - 99.5 wt% (preferably 95 - 99.5 wt%) of isoolefin and about 15 - 0.5 wt% (preferably about 5 - 0.5 wt%) of a multiolefin of about 4 - 14 carbon atoms. These copolymers are referred to in the patents and literature as "butyl rubber"; see, for example, the textbook *Synthetic Rubber* by G. S. Whitby (1954 edition by John Wiley and Sons, Inc.), pages 608 - 609, etc. The term "butyl rubber" as used in the specification and claims includes the aforementioned copolymers of an isoolefin having 4 - 7 carbon atoms and about 0.5 to 20 wt% of a conjugated multiolefin of about 4 - 10 carbon atoms. Preferably these copolymers contain about 0.5 to about 5% conjugated multiolefin. Suitable conjugated multiolefins include isoprene, butadiene, dimethyl butadiene, piperylene. The preferred isoolefin is isobutylene.

Commercial butyl rubber is a copolymer of isobutylene and minor amounts of amounts of isoprene. It is generally prepared in a slurry process using methyl chloride as a polymerization diluent and a Friedel-Crafts catalyst as the polymerization initiator. The methyl chloride diluent offers the advantage that AlCl$_3$, a relatively inexpensive Friedel-Crafts catalyst is soluble in it, as are the isobutylene and isoprene comonomers. Additionally, at the polymerization temperature, the butyl rubber polymer is insoluble in the methyl chloride and precipitates out of solution as fine particles. The polymerization is generally carried out at temperatures of about -90°C to -100°C. (See U.S. Patent Nos. 2 356 128 and 2 356 129.)

In one embodiment the polymerisation process is carried out continuously in a draft tube reactor. Monomer feed and catalyst are continuously introduced to the reactor where an axial flow pump is located. The pump circulates the butyl rubber slurry at high velocity to provide efficient mixing and heat transfer. Polymer slurry containing about 20 - 30 wt% butyl rubber in methyl chloride continuously overflows from the reactor through a transfer line.

Where the desired product is the butyl rubber 'itself, the slurry is fed through the transfer line to a 'flash drum operated at about 140 - 1180kPa (1.38 - 11.58 atm. abs.) and 65 - 75°C. Steam and hot water are mixed with the slurry in a nozzle as it enters the flash drum to vaporize methyl chloride and unreacted monomers which pass overhead and are recovered. The polymer-water slurry is finished by water removal and drying.

The methyl chloride and unreacted monomers which have been flashed off are cooled to condense out most of the water. However, this methyl chloride/hydrocarbon stream must be further dried before it can be processed for recycling. Drying may be accomplished in an alumina drier. The alumina catalyzes the hydrolysis of methyl chloride forming MeOH and HCl. The HCl reacts with some of the isobutylene to form t-butyl chloride.

The methyl chloride and monomers are separated by standard fractionation techniques. Methyl chloride and isobutylene are recovered and the isoprene has been heretofor discarded because the t-butyl chloride concentrates in the isoprene, and is not readily separated from isoprene by standard fractionation techniques. In the practice of this invention, the dried, recovered isoprene is purified for reuse by treatment with activated alumina.

While a wide variety of compounds will remove halides to some extent from a hydrocarbon stream, activated alumina has been found to be a most effective adsorption medium. The term "activated alumina" as used herein means dehydrated alumina of high surface area, such as has been conventionally used in the butyl rubber rubber art among others as a desiccant to remove water from methyl chloride or other streams. Illustrative, examples of commercially available material which meets this description of activated alumina are Kaiser® 201, ALCOA® H-151, and PECHINEY® A.

In one embodiment of this invention, the recovered isoprene stream is contacted with the alumina on a continuous basis using a column packed with alumina. The hydrocarbon is preferably pumped upward through a vertical bed of alumina. It is well within the skill of those in the art to use a modification of this technique for contacting the hydrocarbon scream with the alumina for example, the hydrocarbon can be passed through the column downward or in several vessel in series. Substantially all of the objectionable t-butyl chloride in the liquid isoprene stream can be removed in a single pass through a bed of alumina at a flow rate of one volume of isoprene to one volume of alumina per hour (1 v/v/hr) at 60°C. It is preferred that the purification process be conducted at temperatures above ambient.

Not wishing to be bound by theory, it is believed that the mechanism of halide removal is a chemiadsorption process. That is, in addition to physical adsorption, a chemical reaction takes place converting the organic chloride to other organic compounds, in this case isobutylene, and an inorganic chloride. Hence, while decreasing the temperature improves adsorption rate, increasing the temperature increases the chemical reaction rate. Since the rate controlling factor is the chemical reaction, improved results are seen with increasing temperature.

The capacity of alumina for chlorides in the method of this invention is about 3 wt% chloride as chlorine. Unlike a conventional low temperature adsorption process the alumina cannot be regenerated for the purpose of this invention by simple hot gas regeneration. It is necessary to first caustic treat and water wash the alumina. As a consequence, there is a high energy cost associated with the subsequently required water removal step. Because of the high regeneration cost and other considerations, it is often more convenient to discard the spent alumina.

In the method of this invention, removal of the t-butyl chloride from the hydrocarbon eg isoprene can be accomplished over a wide range of temperatures, such as from -40° C to a temperature below which cracking or polymerisation of the olefinic hydrocarbons or t-BCl occurs, eg 40°C to about 120°C; preferably about 0°C to 80°C. However because of the chemisorption nature of the chloride removal by alumina, it is preferred that the chloride removal be accomplished at an elevated temperature. Thus chloride removal is preferably effected at from 20°C to 120°C or 30°C to 140°C; more preferably at 40°C to 100°C; most preferably 50°C to 80°C, e.g., 60°C to 80°C. The improvement in chloride removal at elevated temperatures is completely unexpected, and contrary to the art recognized advantage in reducing temperature to improve adsorption. As used herein the term "elevated temperature" means a temperature above 20°C. In a particularly preferred embodiment, the t-butyl chloride is accomplished at a temperature of at least 30°C; more preferably, at least 40°C.

Experiments to demonstrate the effectiveness of alumina in the removal of t-butyl chloride from an isoprene stream were conducted both statically and dynamically. In the static test, isoprene contaminated with t-BCl was contacted with alumina in a vial at a fixed temperature for a time sufficient for the alumina to reach a steady state for tBCl removal. Liquid samples were analyzed using gas chromatography (GC) with a 10 foot (3.05m) by 1/8 inch (0.32cm) stainless steel column packed with 5 % SP-2100 on 100/120 mesh Supelco® port.

The dynamic tests were conducted in a simulated liquid solid batch experiment. Isoprene contaminated with t-BCl was circulated at a high flow rate through a static bed of alumina and back to a reservoir. Because of the high circulation rate as compared to the total volume of the system, the per pass conversion of t-BCl over the alumina bed became small and

3

the liquid composition in the entire system was substantially uniform. As a consequence, the system behaved as a uniformly mixed batch reactor. A schematic of the Dynamic Experimental System is shown in the accompanying drawing. Isoprene/t-BCl was circulated from a reservoir 1 by pump 2, through an alumina bed, 3, and back to the reservoir, 1. A sample was withdrawn for GC analysis at sample line 4, run through the GC column, 5, and returned to the system through line, 8 The advantages of the instant invention may be more readily appreciated by reference to the following examples.

**Example 1**

A static isoprene purification run was made using PECHINEY type A high surface area alumina beads (size 2 - 5 mm) which were first heated at 115°C in a vacuum oven at 29.5" (74.93 cm)Hg vacuum (99.9 kPa) for 17 hours, and then cooled at 20"(50.8cm)Hg vacuum (67.7kPa). A t-BCl contaminated isoprene solution was prepared by mixing 4.9g (5.80 ml at 23°C) of t-BCl and 494.6g of distilled isoprene. The solution was estimated to contain about 9,810 ppm of t-BCl, but based on GC analysis the solution contained 11, 210 ppm of t-BCl.

A charge of 1.0 g of $Al_2O_3$ and 20 ml (13.658 g) of the t-BCl/isoprene solution was added to a vial which was then sealed and placed in a constant temperature bath at about -22 to -20°C for 382 hours. GC analysis of the solution after contact with the $Al_2O_3$ showed that the t-BCl concentration dropped from 11,210 to 9,223 ppm. This represents removal capacity of 2.71g of t-BCl per 100g of alumina, or on an equivalent basis, 1.07g of HCl per 100g of alumina. Some isobutylene was found in the solution as a result of the dehydrochlorination reaction of t-BCl on the alumina. The data are summarized in Table 1.

**Example 2**

The test procedure of Example 1 was repeated except that analyses were run only at the beginning and end of the run. GC Analysis after 381 hours of contact time showed a drop in t-BCl concentration from 11.210 to 9.926 ppm. This represents a t-BCl removal; capacity of 2.61g t-BCl/100g alumina, or on an equivalent basis, 1.03g HCl/100g of alumina. Averaging of the results of Examples 1 and 2 gives an average removal capacity at -22°C of 2.66g of t-BCl per 100g of alumina. The results are summarized in Table 1.

**Example 3 to 8**

A series of runs was made employing the same procedure of Example 1 except that Examples 3 and 6 were run at -12 to -10°C and Example 4 and 5 were run at 23 - 25°C. The results, which are summarized in Table 1, showed removal capacity of 3.76g of t-BCl/100g of alumina at -10°C and 9.06g of t-BCl/100g of alumina at 24°C.

It is apparent from the data of Examples 1 - 6 that the removal capacity of alumina for t-BCl increases with temperature. Hence, the mode of t-BCl removal is believed to be by a catalytic reaction rather than by physical adsorption.

**Example 7**

A t-BCl contaminated solution of isoprene was prepared by combining 8.51 of t-BCl, 752.45g of redistilled isoprene, and 1.37g HPLC grade n-heptane which served as an internal standard for GC analysis. The solution has a calculated t-BCl content of 11,163 ppm and 1, 797 ppm n-heptane by weight. A 5 cc sample of this solution was reserved for GC analysis. The remaining 757g were charged to the reservoir of the Dynamic Experimental system (DES). The alumina bed contained 37.5 g of alumina.

At the beginning of the run the system was pressurized to 200 Kpa and the pump was started. The liquid circulated around the system but bypassed the alumina bed unit. The liquid heated up to 60°C at which point it was switched to pass through the alumina bed and the removal of t-BCl began. On line GC analyses were performed periodically. After 67 hours on stream the test was stopped although the t-BCl concentration continued to drop. Analysis showed that the t-BCl content dropped from 11,162 to 554 ppm. That represents an equivalent of 21.42g of t-BCl removal per 100g of alumina at 60°C.

The system was emptied and 960 cc of solution recovered. The system was then washed with heptane. Solution for a second charge was prepared by combining 8.51g of t-BCl, 712.72g of isoprene and 1.37g of n-heptane. The calculated t-BCl was 11,777 ppm and heptane concentration was 1,896 ppm n-heptane. However, as a result of contamination from the first test described above and heptane trapped in the system, the composition of the solution at the beginning of the second 60°C run was 11,290 ppm of t-BCl, 30,620 ppm n-heptane, 335 ppm diisobutylene, 13 ppm of isobutylene and 200 ppm diisoprene.

The second run at 60°C was run under the same conditions as run, but the same alumina was used. After 86 hours of contact with the alumina the GC analysis showed a t-BCl content of 4,354 ppm. Hence, the alumina bed had removed an additional 12.93g of t-BCl per 100g of alumina. The total removal capacity of the alumina was, therefore, 34.35g t-BCl per 100g of alumina, or on an equivalent basis, 13.53g of HCl 100g of alumina at 60°C. The results of Example 7 are summarized in Table 1.

**Example 8**

The experiment of Example 7 was repeated at 32°C using a mixture of 8.51g of t-BCl, 755.12g of isoprene, and 1.37g of n-heptane. The solution was found to have 11,125 ppm t-BCl, 4,123 ppm n-heptane, 17 ppm isobutylene, and 60 ppm of diisoprene by GC analysis. The alumina bed was again packed with 37.5g of fresh alumina. The t-BCl concentration reached a steady state after 96 hours on stream. GC analysis showed that the t-BCl concentration had dropped to 2,900 ppm which is equivalent to 16.78g of t-BCl per 100g of alumina or 6.61g of HCl per 100g of alumina. The results are summarized in Table 1.

**Example 9**

After the test of Example 8 was completed, the system temperature was raised to 43°C and the solution was again recycled through the aluminum bed. The t-BCl level was further lowered from 2,900 ppm to 410 ppm. This represents an additional t-BCl removal of 5.08g of t-BCl per 100g of alumina, and brought the alumina total adsorption capacity at 43°C to 21.86g of t-BCl or 8.619 of HCl per 100g of alumina.

Surprisingly, not withstanding the fact that the alumina had become saturated with t-BCl at 32°C, as evidenced by the achievement of a steady state of t-BCl level, increasing the temperature of the system resulted in additional t-BCl reactivity for the alumina. This is in direct contradiction of the expected results based on conventional adsorption theory and practice, and suggests that the postulated catalytic reaction is in fact the method of t-BCl removal.

**Example 10**

An isobutylene/isoprene solution containing a trace amount of t-BCl was prepared by adding 0.25g t-BCl to 249.75 g of a 75 % isobutylene/25 % isoprene mixture. 10 g of a high surface area alumina (PECHINEY type A) was placed in a 300 ml bomb. After evacuation of the bomb, 101.5 g of the t-BCl contaminated solution was added, and the bomb was sealed. The bomb was shaken for 15 minutes and allowed to stand at 22°C for 70 hours. The composition of the solution before it was contacted with the alumina was:

| Component | Concentration, wt% |
|---|---|
| Isobutylene | 74.1247 |
| Isoprene | 25.7646 |
| t-BCl | 0.0960 |
| Isobutane | 0.0117 |
| Butene-1 | 0.0028 |
| Diisobutylene | 0.0002 |

After 70 hours of contact with alumina, GC analysis of the solution when compared with a GC analysis of the solution prior to contacting it with alumina, showed that the t-BCl peak, which appeared at retention time 16.45 minutes, had disappeared. Hence, the alumina is effective at removing trace amounts of t-butyl chloride from an isobutylene isoprene stream. The GC analysis further showed that there was very little dimer formation during the alumina treatment of the olefinic solution at the lower temperatures.

Based on the data obtained which appears in Table 1, the alumina capacity in t-BCl removal can be represented by the following equation:

$$C = 96{,}375 \, e^{-\frac{2646}{T \, °K}}$$

where C is the t-butyl chloride capacity of the alumina in weight of t-BCl per 100 weight of alumina and T is the temperature of the t-BCl contaminated process stream in degrees Kelvin. Since the capacity of the alumina for t-BCl increases with temperature, the removal phenomenon is not merely an adsorption process, but involves a chemical reaction and therefore is a chemisorption process.

**Table1**

**Removal of tBCl by alumina**

| Type of Test | Exp. No | Rx. Temp. °C | g tBCl/100 g | Alumina | g HCl/100 g | Alumina |
|---|---|---|---|---|---|---|
| Static | 1 | -22 - -20 | 2.71 | | 1.07 | |
| | | | | 2.66 | | 1.05 |
| | 2 | | 2.61 | | 1.03 | |
| Static | 3 | -12 - -10 | | 3.18 | 1.25 | |
| | | | | 3.76 | | 1.48 |
| | 6 | | 4.34 | | 1.71 | |
| Static | 4 | 23 - 25 | 8.68 | | 3.42 | |
| | | | | 9.06 | | 3.57 |
| | 5 | | 9.43 | | 3.71 | |
| Dynamic | 8 | 32 | 16.78 | | 6.61 | |
| Dynamic | 9 | 43 | 21.86 | | 8.61 | |
| Dynamic | 7A | 60 - 62 | 21.42 | | | |
| | | | + | 34.35 | 13.53 | |
| | 7B | | 12.93 | | | |

| Type of Test | Exp No | tBCl Conc. ppm Start | Final | IC4=conc. ppm |
|---|---|---|---|---|
| Static | 1 | | 9,223 | 330 |
| | | 11,210 | | |
| | 2 | | 9,296 | 350 |
| Static | 3 | 11,210 | 8,885 | 576 |
| | 6 | | 8,030 | 644 |
| Static | 4 | | 4,855 | 2,854 |
| | | 11,210 | | |
| | 5 | | 4,306 | 3,073 |
| Dynamic | 8 | 11,125 | 2,900 | |
| Dynamic | 9 | (11,125) | 410 | |
| Dynamic | 7A | 11,163 | 554 | |
| | 7B | 11,290 | 4,554 | |

**Claims**

1. A method of treating an olefinic hydrocarbon contaminated with tertiary butyl chloride, to reduce the contamination level thereof, which method comprises contacting the contaminated hydrocarbon with alumina.

2. A method according to Claim 1 wherein contact is performed at a temperature of from -40°C to 120°C.

3. A method according to Claim 1 or 2 wherein contact is performed at elevated temperature.

4. A method according to Claim 3 wherein the temperature is in the range 30 - 140°C.

5. A method according to Claim 3 wherein the temperature is in the range 20 - 120°C, preferably 40 - 100°C, more preferably 60 - 80°C.

6. A method according to any one of the preceding claims wherein the tertiary butyl chloride is derived from isobutylene.

7. A method according to any one of the preceding claims wherein the olefinic hydrocarbon comprises isoprene.

8. A method according to Claim 7 wherein the olefinic hydrocarbon comprises a mixture of isobutylene and isoprene.

9. A method according to Claim 8 wherein the mixture comprises a major amount of isobutylene and a minor amount of isoprene.

10. A method according to any one of the preceding claims wherein the contaminated olefinic hydrocarbon comprises unreacted dried monomer(s) separated from the product mixture of a methyl chloride slurry polymerisation process for preparing butyl rubber.

11. A method according to Claim 10 wherein the unreacted dried monomer comprises isoprene separated from unreacted dried isobutylene comonomer by fractionation and thereby enriched in said tertiary butyl chloride.

12. A method according to Claim 10 or 11 wherein after said contacting the treated olefinic hydrocarbon is recycled to the polymerisation process.

13. A method according to any one of the preceding claims when carried out on a continuous basis.

14. A process for preparing butyl rubber by methyl chloride slurry polymerisation of a monomer mixture comprising isobutylene and isoprene, wherein the methyl chloride and unreacted monomer are separated from the butyl rubber-containing polymerisation product mixture by flashing in hot water, the recovered methyl chloride-monomer mixture is subsequently dried over alumina, thereby forming tertiary butyl chloride derived from the isobutylene, and the dried methyl chloride is separated from the monomer(s) by fractionation, characterised in that the tertiary butyl chloride is then removed from the unreacted dried monomer(s) by the method according to any one of the preceding claims.

**Revendications**

1. Procédé de traitement d'un hydrocarbure oléfinique contaminé par du chlorure de tertiobutyle pour réduire son niveau de contamination, procédé qui consiste à faire entrer l'hydrocarbure contaminé en contact avec de l'alumine.

2. Procédé suivant la revendication 1, dans lequel le contact est effectué à une température de -40°C à 120°C.

3. Procédé suivant la revendication 1 ou 2, dans lequel le contact est effectué à une température élevée.

4. Procédé suivant la revendication 3, dans lequel la température se situe dans l'intervalle de 30 à 140°C.

5. Procédé suivant la revendication 3, dans lequel la température se situe dans l'intervalle de 20 à 120°C, mieux encore de 40 à 100°C, de préférence de 60 à 80°C.

6. Procédé suivant l'une quelconque des revendications précédentes, dans lequel le chlorure de tertiobutyle est dérivé d'isobutylène.

7. Procédé suivant l'une quelconque des revendications précédentes, dans lequel l'hydrocarbure oléfinique comprend de l'isoprène.

8. Procédé suivant la revendication 7, dans lequel l'hydrocarbure oléfinique comprend un mélange d'isobutylène et d'isoprène.

9. Procédé suivant la revendication 8, dans lequel le mélange comprend une quantité dominante d'isobutylène et une quantité secondaire d'isoprène.

10. Procédé suivant l'une quelconque des revendications précédentes, dans lequel l'hydrocarbure oléfinique contaminé comprend un ou plusieurs monomères séchés n'ayant pas réagi, séparés du mélange obtenu comme produit dans un procédé de polymérisation en suspension dans le chlorure de méthyle pour la préparation de caoutchouc butyle.

11. Procédé suivant la revendication 10, dans lequel le monomère séché n'ayant pas réagi comprend de l'isoprène séparé par fractionnement du comonomère isobutylène séché n'ayant pas réagi, et par conséquent enrichi en ledit chlorure de tertiobutyle.

12. Procédé suivant la revendication 10 ou 11, dans lequel, après la mise en contact, l'hydrocarbure oléfinique traité est recyclé dans le procédé de polymérisation.

13. Procédé suivant l'une quelconque des revendications précédentes, mis en oeuvre sur une base continue.

14. Procédé de production de caoutchouc butyle par polymérisation, en suspension dans le chlorure de méthyle, d'un mélange de monomères comprenant de l'isobutylène et de l'isoprène, dans lequel le chlorure de méthyle et le monomère n'ayant pas réagi sont séparés par une opération flash dans l'eau chaude du mélange constituant le produit de polymérisation contenant du caoutchouc butyle, le mélange récupéré de chlorure de méthyle et de monomère est ensuite séché sur de l'alumine, ce qui forme du chlorure de tertiobutyle dérivé de l'isobutylène, et le chlorure de méthyle séché est séparé du ou des monomères par fractionnement, caractérisé en ce que le chlorure de tertiobutyle est ensuite éliminé du ou des monomères séchés n'ayant pas réagi par le procédé suivant l'une quelconque des revendications précédentes.

**Patentansprüche**

1. Verfahren zur Behandlung eines olefinischen Kohlenwasserstoffs, der mit einem tertiären Butylchlorid verunreinigt ist, um den Grad der Verunreinigung zu verringern, bei dem der verunreinigte Kohlenwasserstoff mit Aluminiumoxid kontaktiert wird.

2. Verfahren nach Anspruch 1 bei dem das Kontaktieren bei einer Temperatur von -40°C bis 120°C durchgeführt wird.

3. Verfahren nach Anspruch 1 oder 2, bis dem das Kontaktieren bei erhöhter Temperatur durchgeführt wird.

4. Verfahren nach Anspruch 3, bei dem die Temperatur im Bereich von 30 bis 140°C liegt.

5. Verfahren nach Anspruch 3, bei dem die Temperatur im Bereich von 20 bis 120°C, vorzugsweise 40 bis 100°C und insbesondere 60 bis 80°C liegt.

6. Verfahren nach einem der vorangehenden Ansprüche, bei dem sich das tertiäre Butylchlorid von Isobutylen ableitet.

7. Verfahren nach einem der vorangehenden Ansprüche, bei dem der olefinische Kohlenwasserstoff Isopren umfaßt.

8. Verfahren nach Anspruch 7, bei dem der olefinische Kohlenwasserstoff eine Mischung von Isobutylen und Isopren umfaßt.

9. Verfahren nach Anspruch 8, bei dem die Mischung eine größere Menge Isobutylen und eine geringere Menge Isopren umfaßt.

10. Verfahren nach einem der vorangehenden Ansprüche, bei dem der verunreinigte olefinische Kohlenewasserstoff nicht-umgesetztes getrocknetes Monomer bzw. nicht-umgesetze getrocknete Monomere umfaßt, die von der Produktmischung eines Methylchlorid-Aufschlämmungspolymerisationsverfahrens zur Herstellung von Butylkautschuk abgetrennt worden sind.

11. Verfahren nach Anspruch 10, bei dem das nicht-umgesetzte getrocknete Monomer Isopren umfaßt, das vom nicht-umgesetzten getrockneten Isobutylen-Comonomer durch Fraktionierung abgetrennt und dadurch mit dem tertiären Butylchlorid angereichert worden ist.

12. Verfahren nach Anspruch 10 oder 11, bei dem der behandelte olefinische Kohlenwasserstoff nach dem Kontaktieren in das Polymerisationsverfahren zurückgeführt wird.

13. Verfahren nach einem der vorangehenden Ansprüche, das auf einer kontinuierlichen Basis durchgeführt wird.

14. Verfahren zur Herstellung von Butylkautschuk durch Methylchlorid-Aufschlämmungspolymerisation einer Isobutylen und Isopren umfassenden Monomermischung, bei dem das Methylchlorid und nicht-umgesetzten Monomer von der Butylkautschuk enthaltenden Polymerisationsproduktmischung durch Entspannen in heißem Wasser abgetrennt werden, die gewonnene Methylchlorid-Monomer-Mischung anschließend über Aluminiumoxid getrocknet wird, dadurch ein sich von Isobutylen ableitendes tertiäres Butylchlorid gebildet wird und das getrocknete Methylchlorid von dem Monomer bzw. den Monomeren durch Fraktionierung abgetrennt wird, dadurch gekennzeichnet, daß das tertiäre Butylchlorid dann von dem nicht-umgesetzten getrockneten Monomer bzw. den nicht-umgesetzten getrockneten Monomeren nach dem Verfahren gemäß einem der vorangehenden Ansprüche entfernt wird.